# FASCICULE DE BREVET EUROPEEN

(11) **EP 2 007 693 B1**
(45) Date de publication et mention de la délivrance du brevet: **28.02.2018**
(21) Numéro de dépôt: 07731848.3
(22) Date de dépôt: 30.03.2007
(51) Int. Cl.: C03C 17/32, C03C 25/62

(54) **PROCEDE DE FONCTIONNALISATION D'UN RENFORT VERRIER POUR MATERIAU COMPOSITE**
VERFAHREN ZUR FUNKTIONALISIERUNG EINER GLASVERSTÄRKUNG FÜR EINEN VERBUNDWERKSTOFF
PROCESS FOR FUNCTIONALIZING A GLASS REINFORCEMENT FOR COMPOSITE MATERIAL

(30) Priorité: 31.03.2006 FR 0651157
(43) Date de publication de la demande: 31.12.2008
(73) Titulaire: Saint-Gobain Technical Fabrics Europe, 73000 Chambéry (FR)
(72) Inventeur: MOIREAU, Patrick, F-73190 Curienne (FR); DURAN, Maxime, F-11570 Cavanac (FR); DENIS, Jean-Baptiste, F-75018 Paris (FR)
(74) Mandataire: Saint-Gobain Recherche
(86) Numéro de dépôt international: PCT/FR2007/051044
(87) Numéro de publication internationale: WO 2007/113444

(56) Documents cités:
- WO-A-01/85635
- WO-A-99/50199
- GB-A- 1 355 769
- GB-A- 2 290 729
- US-A- 5 585 147
- DATABASE WPI Week 198726 Derwent Publications Ltd., London, GB; AN 1987-181665 XP002409341 & JP 62 111493 A (UNITIKA LTD) 22 mai 1987 (1987-05-22)
- DATABASE WPI Week 198343 Derwent Publications Ltd., London, GB; AN 1983-798135 XP002409342 & JP 58 156546 A (NIPPON TELEGRAPH & TELEPHONE CORP) 17 septembre 1983 (1983-09-17)
- DATABASE WPI Week 198907 Derwent Publications Ltd., London, GB; AN 1989-051325 XP002409343 & JP 64 001733 A (MATSUSHITA ELECTRIC WORKS LTD) 6 janvier 1989 (1989-01-06)

## Description

La présente invention est relative à un procédé d'obtention de matériaux composites comprenant une étape d'imprégnation de renforts verriers par une matrice organique, lesdits renforts se présentant indifféremment sous forme de mèches, grilles, tissés, non tissés etc.

Il est connu que les propriétés des matériaux composites dépendent essentiellement de la qualité de l'interface et notamment de l'adhérence entre le substrat verrier et la matrice organique. Les composites sont réalisés actuellement à partir d'un ou plusieurs constituants selon différents procédés de base, au sein desquels on distingue les voies sèches par extrusion et les voies humides par imprégnation.

Par substrat verrier, il est notamment entendu au sens de la présente description, de façon non exhaustive, les tissus de verre, les non-tissés du type complexe, les voiles, les mats, les grilles de verre, notamment pour une application bâtiment.

Une séquence typique des étapes d'un procédé par voie humide comprend une mise en suspension dans un solvant aqueux du polymère constitutif de la matrice et du substrat ou renfort verrier, une filtration sous vide et une mise en forme du produit, par exemple par calandrage. L'eau résiduelle est ensuite éliminée sous étuve. Ainsi, dans le cas ou la fabrication du matériau composite est effectuée par voie humide, la qualité de l'imprégnation (vitesse, enrobage homogénéité de l'enrobage, etc.) est un facteur primordial pour le choix du procédé le plus performant. Ce facteur conditionne notamment les performances des composites finalement obtenues et la satisfaction du client qui en résulte.

Plus encore, un défaut visible d'imprégnation, même minime et ne perturbant pas les performances globales du matériau, peut donner un a priori défavorable sur le produit.

Un autre facteur bien évidemment décisif est le coût économique de ces procédés, dès lors qu'ils sont développés à une échelle industrielle.

On connaît dans la littérature de très nombreuses publications traitant de la fabrication des composites et plus particulièrement des problèmes d'imprégnation des fibres de renfort.

On sait ainsi maintenant qu'il est notamment possible de jouer sur différents facteurs pour améliorer l'imprégnation, par exemple
- sur la structure du renfort, par modification de la porosité du matériau ou de l'orientation des fibres,
- sur la chimie de l'ensimage ou des liants, par modification de la tension de surface des fibres, du gonflement de l'ensimage, la rugosité de surface, la redissolution d'espèces chimiques etc.,
- sur la chimie des résines, des suspensions ou des émulsions imprégnant le matériau fibreux en modifiant par exemple la tension de surface du liquide ou sa rhéologie,
- sur les paramètres du procédé par modification des temps d'imprégnation, de la pression, de la température, du taux de dilution, etc.

Cependant, le défaut d'adhésion entre le renfort verrier et la matrice organique est essentiellement le résultat d'une mauvaise mouillabilité de la fibre ensimée constitutive du renfort dans la solution d'imprégnation. Le défaut d'adhésion peut ainsi être directement lié à la réactivité chimique de la surface du renfort verrier par la solution utilisée.

Il est connu que les renforts verriers pour matériaux composites, qu'ils soient sous forme de mèches, grilles, tissés, non tissés ou autres, nécessitent pour leur fabrication, plus particulièrement lors du fibrage des fibres de verre les constituant, l'utilisation de compositions organiques complexes. Ces compositions assurent à la fois la liaison entre les filaments de verre dans la fibre ainsi qu'entre le renfort et la matrice et jouent un rôle lubrificateur et protecteur contre l'abrasion des filaments entre eux.

Les compositions organiques jouant un tel rôle sont en général regroupés dans le métier sous le terme d'ensimage.

Il est généralement admis que les problèmes d'imprégnation rencontrés entre les renforts verriers et la matrice organique résultent d'une mauvaise compatibilité entre l'ensimage enrobant les renforts et la solution utilisée pour effectuer ladite imprégnation.

Certaines solutions rapportées dans l'art antérieur pour améliorer les techniques d'imprégnation, notamment dans le domaine des textiles pour application électronique ou pour des applications matériaux hautes performances, préconisent une étape d'élimination de l'ensimage présent dans les renforts et une étape de traitement visant à déposer, à la place de l'ensimage, un composé organique appelé dans le métier agent de couplage. Le rôle de cet agent de couplage est d'améliorer l'adhésion entre le renfort verrier et la matrice organique. Comme exemple d'agent de couplage les plus utilisés, on peut citer les organosilanes.

Selon un premier exemple, l'étape d'élimination de l'ensimage peut être effectuée par un traitement thermique du renfort verrier à une température d'environ 400°C pendant quelques heures, voire quelques dizaines d'heures. Il a été observé en effet que le traitement devait être prolongé jusqu'à l'élimination total de l'ensimage, la persistance d'une quantité même très faible d'ensimage entraînant une très forte décroissance des propriétés d'adhésion entre le renfort et la matrice dans le produit composite. Ce traitement présente ainsi des inconvénients majeurs liés à sa très faible productivité et à son coût énergétique.

Alternativement, le brevet US 5,585,147 décrit un procédé de traitement de surface d'un tissu de verre dans lequel une élimination totale de l'ensimage est assurée par un plasma atmosphérique d'un gaz préalablement chauffé à une température comprise entre 100 et 500°C, avant une étape de mise en contact avec un agent de couplage constitué par un organosilane.

Selon une autre méthode connue, le renfort verrier peut être traité au moyen d'une décharge électrique filamentaire dans l'air à pression atmosphérique, du type CORONA.

De tels traitements sont bien connus, notamment de la demande de brevet EP 1 044 939 A1 se rapportant aux traitements de surface de fibres de renforcement ou de la demande JP 2-166129 se rapportant à une base de tissu de verre.

Les documents GB 2290729 et JP62111493 décrivent des procédés similaires comprenant une étape intermédiaire de dépôt d'un organosilane pour améliorer la compatibilité de la fibre de verre et de la matrice organique, après élimination par plasma de l'ensimage.

Le document JP 58156546 se rapporte au domaine des fibres optiques. Le traitement plasma a pour but d'éliminer les microfissures à la surface des fibres pour en augmenter la résistance mécanique. Un procédé similaire est décrit dans la demande GB 1,355,769.

Le document JP 64001733 décrit un procédé dans lequel un plasma est utilisé pour l'élimination de l'ensimage présent sur les fibres pour permettre l'étape de fibrage, avant l'imprégnation dans la matrice.

Le document WO 99/50199 décrit une méthode de fonctionnalisation de la surface des fibres de verre pour déposer une quantité accrue de silane, c'est-à-dire d'agent de couplage avec la matrice. Le renfort verrier doit être propre c'est-à-dire exempt d'ensimage avant le traitement plasma utilisé.

Le document WO 01/85635 décrit un procédé très éloigné de la présente invention dans lequel, lors d'une première étape, un plasma est utilisé pour nettoyer la surface des fibres et générer sur la surface verrière des radicaux, et dans lequel lors d'une seconde étape, une couche d'hydrocarbures est formé sur lesdits radicaux.

Un traitement de surface réalisé à l'aide d'une décharge électrique type « CORONA » est caractérisé par un régime de décharge électrique de type filamentaire à pression atmosphérique dans l'air.

En effet, dans la majorité des gaz de type industriel (argon, air, azote...), leur claquage à pression atmosphérique, qui est en fait une transition vers un régime conducteur du gaz, est initié par un grand nombre de filaments indépendants ou micro-décharges dont les caractéristiques sont notamment une durée de vie inférieure à 10⁻⁹s, un rayon moyen inférieur à 100 µm, et une densité de courant comprise entre 100 à 1000 A/cm². Les micro-décharges s'allument et s'éteignent aléatoirement sur toute la surface des électrodes, dont l'une au moins peut être recouverte d'une barrière diélectrique. Dans ce régime filamentaire, les matériaux à traiter sont directement mis au contact de la décharge électrique, c'est-à-dire entre les deux électrodes (traitement in situ), le traitement de surface des matériaux se faisant globalement de manière plus ou moins homogène. En revanche, localement, les transformations induites par ce type de traitement par décharges filamentaires sont très hétérogènes. Ainsi, une portion de surface du matériau ayant subit une série de micro-décharges sera beaucoup plus modifiée, voire dans les cas extrêmes attaquée et dégradée qu'une autre portion qui n'en aura pas subit, même sur des bases inorganiques comme le verre. En outre, la décharge électrique de type « CORONA », du fait de son intensité, a tendance à créer, au niveau des zones d'impact des micro filaments avec la surface du renfort, des zones de fragilisation (échauffement local, amorce préférentielle des fissures) qui diminuent les propriétés mécaniques du produit composite final.

Il a de plus été démontré que les conséquences du traitement CORONA sur la portion de surface du substrat ne sont généralement pas pérennes dans le temps.

Les décharges électriques filamentaires du type CORONA, bien que permettant de traiter efficacement la surface d'un renfort, montrent en outre d'autres inconvénients majeurs :
- le traitement est souvent limité au traitement de structures bidimensionnelles 2D , la configuration plan - plan des électrodes étant adaptée à une géométrie 2D, les tissus passent dans la décharge et ils peuvent être traités sur les faces,
- le traitement filamentaire n'est pas homogène et difficilement contrôlable sachant que son efficacité dépend beaucoup du pourcentage d'humidité relative de l'air, par exemple,
- le traitement filamentaire peut dégrader, par un échauffement local ou par une amorce de rupture, la surface traitée et mener à une perte des propriétés mécaniques de la fibre,
- le traitement filamentaire peut déposer une quantité de charges électriques importante à la surface du renfort, susceptible ensuite de perturber l'étape d'imprégnation ultérieure,
- la chimie du traitement est limitée à l'oxydation des substrats.

L'objet de la présente invention est de fournir un nouveau procédé d'obtention d'un matériau composite comprenant notamment une étape de traitement du substrat verrier avant son imprégnation, le procédé étant plus simple et plus économique que ceux précédemment décrit.

Plus précisément, l'invention se rapporte à un procédé d'obtention d'un matériau composite comprenant un substrat ou renfort verrier dans une matrice organique ou minérale se présentant sous forme de mèches, grilles, voiles, tissés, non tissés ou similaires selon les revendications ci-jointes.

Le procédé selon l'invention comprend au moins les étapes suivantes :
- traitement de surface du renfort verrier par un plasma homogène d'un mélange gazeux, dans une atmosphère gazeuse contrôlée d'oxydation ou de nitruration de l'ensimage organique présent à la surface du renfort verrier,
- imprégnation du renfort ainsi fonctionnalisé par ladite nitruration ou ladite oxydation par une émulsion ou une suspension aqueuse de la matrice ou directement par la matrice.

Le plasma homogène est généralement mis en oeuvre à une pression inférieure à la pression atmosphérique, de l'ordre de 10⁻⁵ bar et de préférence sensiblement à la pression atmosphérique. De préférence, la température du mélange gazeux est inférieure à 90°C. Par exemple, le mélange gazeux peut comprendre ou être constitué d'oxygène.

Selon un autre mode de réalisation, le mélange gazeux comprend ou est constitué de N₂ ou d'un mélange de N₂ et d'un gaz réducteur du type H₂ ou NH₃ ou encore comprend ou est constitué de NH₃.

L'invention se rapporte également au matériau composite susceptible d'être obtenu par le procédé tel que précédemment décrit.

Dans le matériau composite selon l'invention, la matrice organique est choisie dans le groupe constitué par les matrices thermoplastiques ou thermodurcissables, par exemple dans le groupe constitué par les polyvinylalcool, polyvinylacétate polyvinylchlorure, polytétrafluoroéthylène et les copolymères dérivés de ces polymères tels que Polyvinylacétate-polyéthylène.

Dans le matériau composite selon l'invention, le renfort verrier est de préférence choisi parmi les tissus de verre, les non-tissés du type complexe, les voiles, les mats, les grilles de verre, notamment pour une application bâtiment, les fils unitaires ensimés ou préalablement transformés, les fils composés, le type de verre pouvant être choisi parmi les verres E, R, ECR, S, ou encore toute composition verrière connue pour ses propriétés anticorrosions vis-à-vis de conditions acides ou basiques, et/ou de haute résistance mécanique.

Le présent procédé peut être mis en oeuvre de différentes manières dont deux exemples sont donnés ci-après :
Selon un premier mode de réalisation, on effectue la succession des étapes suivantes :
   1°) Fabrication du renfort avec ensimage (ex : mèches, grilles, tissés, non tissés)
   2°) Préparation de la surface par un traitement plasma tel que précédemment décrit,
   3°) Imprégnation du renfort traité.

Selon un autre mode de réalisation, on effectue la succession des étapes suivantes :
1°) Fabrication des renforts élémentaires (ex : mèches)
2°) Préparation de la surface des renforts élémentaires par le traitement plasma selon l'invention,
3°) Fabrication du renfort complexe à partir des renforts élémentaires traités (par exemple sous forme de tissés, non tissés, voiles, grilles),
4°) Imprégnation dans une matrice organique.
Selon ce mode, les étapes 3 et 4 peuvent être interverties. Dans ce dernier cas, on prévoira de préférence une cinquième étape de collage par calandrage pour améliorer encore l'adhérence.

Il est bien évident que l'invention pourrait être mise en oeuvre selon d'autres modes aisément accessibles à l'homme du métier, qu'il serait fastidieux de tous les reporter dans la présente description. L'ensemble des modes possibles de mise en oeuvre du présent procédé est bien entendu compris dans le cadre de la présente invention.

D'un point de vue technique, la décharge électrique plasma homogène selon l'invention est amorcée entre deux électrodes soumises à une différence de potentiel appropriée, dans une atmosphère contrôlée d'un mélange de gaz choisi. Suite à l'application du champ électrique, le gaz s'ionise (principe d'avalanches). Les électrons et les ions créés acquièrent de la vitesse et interagissent avec les particules neutres du gaz. En fonction de leur énergie cinétique, il en résulte la création de nouvelles particules ionisées et d'espèces chimiques dans un état excité.

Les espèces actives créées au sein d'un gaz ionisé sont en principe les électrons, les ions positifs et négatifs, les atomes et molécules métastables, les espèces disposant d'énergie cinétique ou vibrationnelle, les radicaux libres, les photons. Toutes ces espèces sont susceptibles d'interagir avec la surface des matériaux. Leur action est variable en fonction du type de décharge électrique et des conditions expérimentales qui déterminent notamment leur nombre, leur répartition et leur énergie.

Dans le cas d'un traitement de surface selon l'invention, la distribution en énergie des électrons est centrée sur quelques électrons volts, typiquement entre 0,5 et 100 eV. Les espèces précédemment citées entrent en contact avec la surface du renfort à traiter, c'est-à-dire principalement avec l'ensimage organique utilisé pour le fibrage du renfort. Chaque espèce est susceptible d'engendrer des modifications chimiques plus ou moins profondes de l'ensimage en fonction de son énergie et de son libre parcours moyen dans le solide.
Sans que cela puisse être compris ou lié comme une quelconque théorie, les effets surprenants observés par la demanderesse sur la vitesse et/ou les qualités d'imprégnation des renforts traités selon l'invention pourrait s'expliquer par une activation de surface des renforts, liée à différents changements structuraux tels qu'une réticulation ou une fonctionnalisation (notamment par greffage de nouvelles fonctions chimiques) du substrat, voir à une modification de la rugosité, de la physico-chimie, de la charge électrique ou de la mécanique (densité, réticulation) des surfaces organiques traitées.

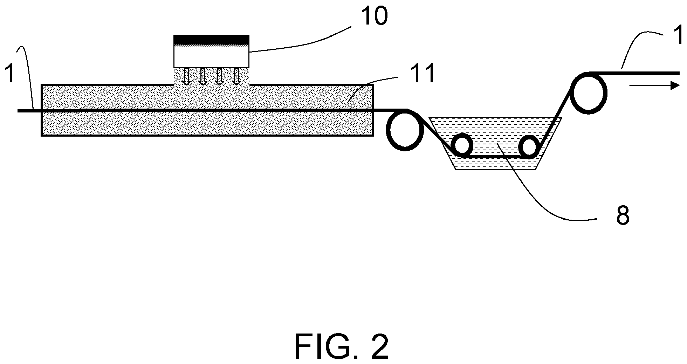

Le présent procédé présente les avantages suivants:
- le procédé de fonctionnalisation de la surface du renfort est effectué sans l'assistance de solvants,
- la configuration des traitements plasma homogène peut directement être adapté en ligne avec le mode de fabrication des renforts enduits,
- le traitement plasma peut être réalisé à pression atmosphérique,
- le traitement plasma est homogène quelle que soit la forme et les dimensions du renfort à traiter (2D, 3D),
- Le traitement plasma selon l'invention n'altère pas les propriétés mécaniques des renforts,
- La chimie de surface du traitement plasma est modulable, notamment en fonction de la nature de l'ensimage et de la matrice organique,
- la charge de surface est moindre, en particulier avec les traitements 3D selon le mode post-décharge illustré par la figure 2 (plasma déporté).
- le traitement plasma selon l'invention limite les problèmes de vieillissement du renfort traité observés auparavant sur les renforts traités par décharges électriques de type « CORONA ».

Parmi les nombreuses variantes possibles caractérisant le traitement plasma selon l'invention on peut citer :
- les traitements plasma par pulvérisation cathodique assistée magnétron ou/et du type IBS (Ion Beam Source),
- le greffage par plasma de l'ensemble des fonctions connues à base d'oxygène ou d'azote,
- les dépôts d'espèces chimiques assistés par plasma du type oxydes ou nitrures, en particulier SiOₓC_{y}, SiOₓNₓC_{z}, AlOₓ,TiOₓ,TiNₓ et leurs mélanges.

Les ensimages susceptibles d'être modifiés en surface par le présent procédé sont par exemple les ensimages pour composites en masse, telles que ceux utilisés dans les applications automobiles, devant assurer une liaison très forte avec la matrice organique sont dans le but d'obtenir de très fortes valeurs de propriétés mécaniques (composite époxy certains thermoplastiques type PA haute température) ou de vieillissement en assurant des liaisons chimiques moins sensibles aux réactions hydrolytiques (applications en milieu humide ou corrosif). Les compositions de ces ensimages sont surtout des mélanges d'émulsions à base de polymères variés (Epoxy, Polyester, Polyuréthanne, Polyvinylacétate, etc.) associés à des agents de couplage (silanes,...) et des agents de mise en oeuvre (lubrifiants, tensioactifs, etc.).
Les émulsions ou suspensions utilisable selon la présente invention sont par exemple les solutions aqueuses de PVA, PVC, SBR, acrylique, PTFE, Silicone **etc.**
Selon l'invention on peut utiliser comme renfort des produits ouverts de type grille ou non tissés à larges mailles (Laid scrims) destinés aux applications bâtiment (façade cladding), renfort du papier, vêtement de protection, greffage phénolique (meules).
Le dépôt du revêtement sur le renfort traité peut être effectué selon toute méthode connue d'imprégnation parmi lesquelles les procédés dits roll, dip, spray, selon les termes anglais employés dans le métier.

Selon la méthode d'imprégnation utilisée, les produits de base peuvent être également de type tissus fermés, de mats ou de voiles ou de fils coupés (thermoplastiques, automobile) ou de produits destinés à des applications de type enroulement filamentaire (cuves, tuyaux, etc.).

Les applications potentielles de la présente invention sont multiples. De fait, les fibres de verre transformées ou non sous forme de produits élaborés sont destinées à renforcer principalement des matrices organiques polymériques. Dans tous les cas, la compatibilité et la qualité de l'imprégnation par la matrice à renforcer sont deux facteurs clés influençant de manière très importante les propriétés mécaniques et la durabilité du composite obtenu.

Parmi les nombreuses applications potentielles, on peut citer, de manière non exhaustive :
1 - Les composites qui nécessitent une imprégnation par une matrice organique pour fabriquer des composites « en masse ». Ce sont les composites de type plan (2D) ou volumique (3D) tels que les composites renforcés par des tissus, des complexes, des non tissés tels que des voiles, principalement à base de résines polyester ou époxy. Des procédés tels que les techniques d'infusion peuvent tirer un grand avantage d'une amélioration de l'imprégnation et en particulier de la cinétique de mouillage du renfort. Les principaux domaines d'utilisation sont les transports, les sports et loisirs, le bâtiment (tuyauteries renforcées, certains bardages...), des éléments de génie civil (ponts routiers ou piétonniers, mâts, éléments de structure, ...),
2 - Des systèmes de type SMC/BMC, renforcés par des fils coupés longs ou des produits de type mat Unifilo® peuvent aussi être concernés. L'imprégnation sous forme d'une pâte par la matrice est aussi un point clé pour l'aspect final du produit que pour les propriétés mécaniques. Les applications se trouvent principalement dans le domaine des transports, mais d'autres applications assez nombreuses existent dans les systèmes électriques, les équipements sanitaires,....
3 - Les produits tissés ou non tissés destinés au renfort de matrices particulières et aux propriétés de surface peu compatibles avec une bonne adhésion sur des supports verriers même préalablement traités par un ensimage. Il s'agit en particulier de matrices telles que le PVC, le PTFE et tous les copolymères dérivés de ces 2 polymères. Les applications sont diverses parmi lesquelles les bandes transporteuses pour l'agroalimentaire, les protections contre les agressions chimiques (équipements militaires NBC en PTFE), les couvertures de stade....
4 - Les grilles de verre tissées ou non tissées à deux niveaux différents de leur procédé de fabrication et de leur utilisation :
   Sur les grilles écrues, la modification de la surface des filaments et plus particulièrement des propriétés de l'ensimage les recouvrant, peut permettre une meilleure imprégnation à coeur et une meilleure adhésion entre la matrice et les filaments de verre (amélioration de l'interface). Cela est particulièrement important pour des applications nécessitant une bonne résistance à l'attaque alcaline telles que les applications, selon les termes anglais utilisés dans le métier : « façade cladding », pour les joints de panneaux en ciment ou en plâtre, le « cement board », ou des applications requérant une bonne résistance à l'humidité telles que les applications « roofing, insect screen » etc. Sur les grilles déjà revêtues d'un revêtement, la modification de surface peut permettre une meilleure compatibilité ou adhésion avec les produits à renforcer notamment dans les applications bâtiment (ciments souvent modifiés par des polymères tels que des acryliques), ou des applications dans le domaine du papier (papiers renforcés, papier d'essuyage...).

Ces exemples représentent quelques procédés et applications parmi les principaux domaines et marchés de la fibre de verre de renfort. Les applications et les procédés sont très nombreux et il est impossible de les citer tous dans le cadre de la présente description. Néanmoins cette technologie plasma de modification de surface a l'avantage de présenter une grande souplesse et une grande variété quant aux types de traitements applicables et de ce fait, peut être applicable à une partie importante des produits de renfort, en particulier les produits ayant déjà subit une première étape de transformation tels que les tissus, les grilles, les mats etc.

D'autres caractéristiques et avantages de l'invention apparaîtront au cours de la description non limitative qui suit de différents modes de réalisation de l'invention, illustrée par les figures 1 à 4 parmi lesquelles :
- la figure 1 est une vue schématique d'une installation pour la mise en oeuvre du procédé de traitement de surface conforme à l'invention,
- la figure 2 illustre l'intégration d'une variante de réalisation comprenant la mise en oeuvre d'une installation à plasma déporté,
- La figure 3 est un oscillogramme d'un régime filamentaire,
- La figure 4 est un oscillogramme d'une décharge en régime homogène.

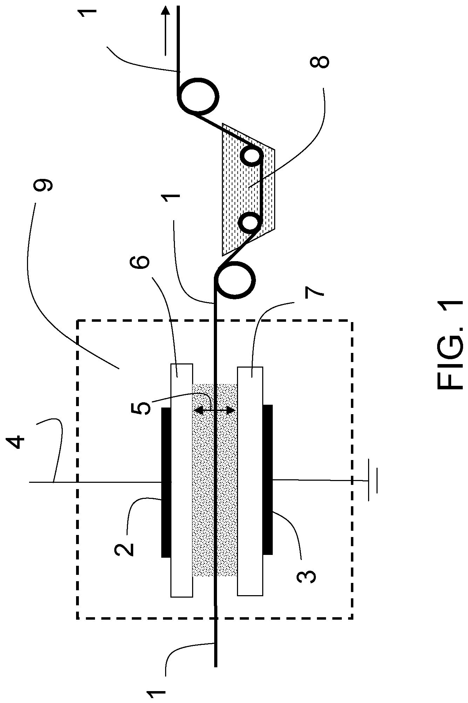

Le matériau de renforcement à base verrière 1 est dirigé au sein d'une zone de traitement de surface, dans une installation générant un plasma homogène adaptée à la mise en oeuvre du procédé. Cette installation, illustrée schématiquement par la figure 1, est d'un type connu.

Lorsque l'atmosphère contrôlée de gaz est à basse pression, elle comporte une enceinte représentée par le repère 9 sur la figure 1. L'enceinte 9 est étanche à l'égard de l'environnement extérieur et peut ainsi être le siège d'une atmosphère contrôlée en composition et en pression.

Elle dispose à cet effet d'une pluralité de conduites destinées aux apports et aux évacuations de ladite atmosphère (non représentées sur la figure 1).

Le dispositif plasma comprend deux électrodes 2 et 3, respectivement reliées aux bornes d'un générateur 4 de tension à fréquence variable. Les électrodes positionnées en regard l'une en face de l'autre délimitent entre-elles un volume de traitement 5 adapté pour le passage du renfort verrier 1 qui peut se présenter indifféremment sous forme de mèches, de grilles, de tissés, de non tissés ou autres.

Selon une autre caractéristique de l'installation lorsque l'atmosphère contrôlée de gaz est à la pression atmosphérique, l'enceinte 9 est facultative mais chacune des électrodes 2, 3 est revêtue par une couche de diélectrique 6, 7 dirigée vers le volume de traitement 5. Dans l'exemple de réalisation représenté en figure 1, chaque couche de diélectrique 6, 7 est par exemple à base d'alumine et est séparée d'une épaisseur comprise entre 0,1 à 20 mm, préférentiellement comprise entre 1 et 6 mm.

Selon le mode de réalisation de l'invention dans lequel l'atmosphère contrôlée est constituée d'un gaz oxydant ou nitrurant à basse pression, typiquement de l'ordre 10⁻⁵ bar. Dans le mode où un plasma à la pression atmosphérique est utilisé, le gaz est constitué majoritairement d'azote, seul ou en mélange avec des espèces réductrices (NH₃, H₂, etc.), ou d'un gaz neutre hélium ou argon, en mélange avec une ou plusieurs espèces oxydantes (notamment obtenues par ionisation de O₂, CO₂, H₂O, etc.) .

En appliquant une tension adéquate aux bornes des électrodes 2, 3, en l'occurrence dans cet exemple une tension alternative de l'ordre de quelques kV et selon une fréquence variant du kHz à quelques dizaines de MHz, en présence de ladite atmosphère contrôlée, une décharge électrique homogène est initiée.

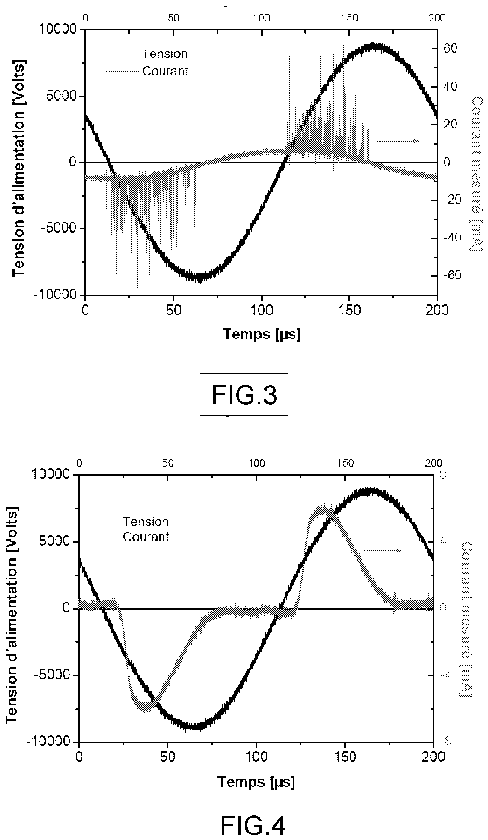

On rappelle qu'au sens de l'invention, et plus généralement, une décharge est dite homogène par opposition à une décharge CORONA lorsqu'il n'est pas possible, à l'échelle macroscopique et microscopique, d'apercevoir entre les électrodes la présence d'arc ou de filaments, de micro-décharges, entre deux électrodes soumises à une différence de potentiel, dans une atmosphère contrôlée d'un mélange de gaz tel que précédemment défini, et à pression atmosphérique. On peut mettre en évidence la nature du régime par un oscillogramme tension/courant (cf. figures 3 et 4). Selon l'invention, la présence d'une décharge homogène confinée entre les électrodes 6 et 7, au niveau de la zone de traitement 5, dans une atmosphère plasma oxydante ou nitrurante, permet avantageusement d'activer chimiquement la surface du renfort verrier ensimé, en particulier de la préparer à l'étape d'imprégnation, schématiquement illustré par l'immersion du renfort traité dans un bain 8 d'une solution aqueuse d'une émulsion ou d'une suspension de la matrice organique. Sans sortir du cadre de l'invention, l'imprégnation du renfort verrier peut également être effectuée par immersion directe dans la matrice, c'est à dire sans étape intermédiaire telle que le dépôt d'un agent de couplage.

Selon un autre mode de réalisation possible permettant la mise en oeuvre du procédé objet de l'invention et illustré par la figure 4, on utilise un dispositif 10 générant un plasma homogène déporté ou soufflé, celui-ci est injecté et rempli un logement 11 de section approprié au sein duquel le renfort sous ses différentes formes possibles, chemine et subit le traitement de fonctionnalisation de sa surface.

Les portions de surface de ces fibres dont la surface a été chimiquement activée par le traitement de fonctionnalisation objet de l'invention sont ensuite imprégnées dans une solution aqueuse 8 comprenant la matrice organique, sous forme d'émulsion ou de suspension.

Les avantages de la présente invention sont illustrés par les exemples qui suivent, non limitatifs de la présente description.

### Exemple 1 :

Dans cet exemple, on a mesuré l'influence d'un traitement plasma homogène selon la présente invention sur un tissu de verre servant de renfort dans un matériau composite. Le tissu est formé à partir d'un fil de base de 9 µm /68 tex/Z20. Le tissu, de masse volumique 209 g/m², comprend 173 fils/10cm en chaîne et 126 fils/10cm en trame. L'ensimage utilisé est un ensimage classique du type textile à base d'amidon, en mélange avec des lubrifiants.

Le tissu de verre est soumis à un traitement plasma homogène dans un réacteur plasma comprenant une enceinte ou chambre sous vide incluant deux électrodes plates métalliques, connectées à un générateur radiofréquence (réacteur à couplage capacitif à électrodes parallèles). L'air contenu dans la chambre est aspiré par une pompe turbo-moléculaire couplée à une pompe rotative, pour obtenir une pression finale de l'ordre de 5.10⁻⁵ bar. Le substrat à traiter est placé sur l'électrode inférieure. Le gaz plasmagène est injecté dans le réacteur au moyen d'un régulateur de débit massique. Le substrat est maintenu à température ambiante (environ 21°C). La formation du plasma est obtenue par application d'un potentiel rf de 13,56 MHz à l'électrode motrice.

Le gaz utilisé est soit de l'oxygène sous une pression de 60 mTorr (soit environ 8 Pascals), soit du NH₃ sous une pression de 150 mTorr (soit environ 20 Pa). Lorsque le gaz est l'oxygène, l'électrode motrice est alimentée à une puissance de 280 Watts. Lorsque le gaz est NH₃, l'électrode motrice est alimentée à une puissance de 190 Watts. La durée du traitement plasma est de 5 minutes dans tous les cas.

Les renforts ainsi obtenus sont soumis à un test de résistance à la traction suivant la norme NF-EN-ISO 13934-1, mesurant les propriétés des tissus en traction par la méthode des bandes (détermination de la force maximale et de l'allongement maximal du tissu).

Les résultats obtenus sont reportés dans le tableau 1 :

**Tableau 1**

| | Résistance à la traction (Newtons) |
|---|---|
| Référence (sans traitement) | 990 ± 40 |
| Traitement Plasma O2 | 1075 ± 90 |
| Traitement Plasma NH3 | 1050 ± 90 |

La comparaison des résultats montre que le traitement plasma n'altère pas les propriétés mécaniques du renfort et par suite celles du matériau composite final obtenu après imprégnation dans la matrice organique.

### Exemple 2 :

Sur différents tissus de verre du même type que ceux de l'exemple 1, ayant subi un traitement plasma oxydant ou réducteur identique à celui décrit dans l'exemple 1, la composition chimique de la couche superficielle a été mesurée par XPS sur une épaisseur de 5 nm. Les compositions obtenues peuvent être comparées grâce au tableau 2 à celle d'un tissu de renfort n'ayant pas subi le traitement plasma de fonctionnalisation de la surface selon l'invention.

On observe que la teneur en silicium dans la couche superficielle est sensiblement identique à celle du tissu non traité. De même, la teneur en carbone reste très majoritaire sur la couche superficielle du tissu traité, ce qui montre que seules les couches atomiques les plus externes du tissu ont été modifiées par le traitement plasma.

**Tableau 2**

| | Carbone | Azote | Oxygène | Fluor | Silicium |
|---|---|---|---|---|---|
| Référence (sans traitement) | 74 | 1 | 21 | 0 | 4 |
| Traitement Plasma O2 | 59 | 0,5 | 3 | 1,5 | 8 |
| Traitement Plasma NH3 | 70 | 8 | 17, 5 | 0,5 | 4 |

### Exemple 3 :

La vitesse d'imprégnation d'un tissu de renfort obtenu par traitement plasma homogène sous atmosphère réduite d'oxygène conformément à l'exemple 1 a été évaluée par la mesure du temps nécessaire pour la pénétration totale d'une goutte d'eau de 3 µl dans le tissu, au moyen d'un dispositif comprenant une caméra reliée à un ordinateur de mesure. Le temps nécessaire à une absorption totale est de 22,5 secondes pour le tissu non traité alors qu'il est de 2,4 secondes pour le tissu préalablement traité par le plasma homogène.

### Exemple 4 :

Les propriétés d'imprégnation, par une suspension aqueuse de PTFE (polytétrafluoroéthylène), du tissu de renfort traité par traitement plasma homogène sous atmosphère réduite d'oxygène conformément à l'exemple 1, ont été évaluées.

Dans ce but, les phénomènes liés à l'imprégnation capillaire des fils et tissus de verre sont mesurés au moyen d'une balance Wilhelmy permettant d'évaluer la vitesse d'imprégnation capillaire ainsi que la masse de liquide retenue par le tissu ou le fil.

Le dispositif de la balance Wilhelmy comprend une balance de précision (0,1 mg) sous laquelle il est possible de fixer un fin crochet métallique relié au plateau de mesure. Ce crochet permet de suspendre un échantillon de mèche de fibres de verre ou un échantillon de tissu.

Les échantillons sont préparés en découpant des bandes de 5 cm de longueur et de 0,5 cm de large dans les tissus de renfort. Ces échantillons sont prélevés dans des bobines de tissu conservées pendant 24 heures à 20°C ± 3°C à une humidité résiduelle de 50% ± 5%.

Les bandes sont dans un premier temps suspendus à la balance Wilhelmy au moyen du crochet situé en dessous. Le tissu suspendu à la balance au moyen du crochet est ensuite mis en contact avec un liquide.

La montée capillaire du liquide dans le fil ou le tissu mesurée par la balance est enregistrée en fonction du temps. Lorsque le tissu est arrivé à saturation de liquide, il est retiré et sa masse est relevée. La masse mesurée correspond à la somme de la masse du tissu sec et de celle du liquide imprégné.

Les résultats sont exprimés sous la forme de courbes donnant la masse de liquide imprégné en fonction de racine du temps. La portion linéaire de cette courbe permet alors d'en tirer une valeur de pente k. Cette valeur exprimée en g.s^{-0,5}, selon la loi de Washburn, est caractéristique de la vitesse d'imprégnation du liquide dans le fil ou le tissu. Une valeur de k élevée peut être alors associée à une imprégnation capillaire rapide. La valeur de la masse de liquide retenue est en outre caractéristique des qualités d'imprégnabilité ou de mouillabilité, c'est-à-dire de compatibilité chimique entre le renfort et la solution.

Les résultats obtenus sont reportés dans le tableau 3 permettent d'apprécier l'augmentation très sensible de la vitesse d'imprégnation d'un tissu traité selon la présente invention.

On constate notamment que la masse de liquide retenue augmente sensiblement après traitement plasma. Cette augmentation est accompagnée d'un accroissement significatif de la vitesse d'imprégnation du renfort dans le solvant aqueux (multiplication par un facteur 6).

**Tableau 3**

| | k (g.s^{-0,5}) | Masse de liquide (grammes) |
|---|---|---|
| Référence (sans traitement) | 0,43 | 14,2 |
| Traitement Plasma O2 | 3,25 | 19,7 |

### Exemple 5:

Une mèche de renfort de verre, composée d'une fibre de 2400 tex (2000 filaments, 2,4 µm) et incorporant un ensimage du type époxy incluant des agents de couplage amine et méthacrylate et des lubrifiants du type cire et alkylbenzène, est utilisée selon cet exemple. Ladite mèche a été traitée selon l'invention par une source plasma déportée à pression atmosphérique selon les principes décrits précédemment en relation avec la figure 2, avec un gaz plasmagène N₂. La source plasma utilisée est commercialisée par la société Acxys technologie sous la référence UL120.

La mèche de verre avance dans la zone plasma à une vitesse de 2 mètres par minute. L'azote est injecté dans le réacteur tubulaire de longueur 1 m à un débit de 250 litres par minute. La source plasma Acxys est activée à une puissance de 2000 W.

La capacité d'imprégnation de la mèche ainsi traitée dans un solvant aqueux est évaluée par plongée de l'extrémité de la mèche dans un récipient d'eau contenant un pigment coloré, en comparaison d'une mèche non traitée. Au bout de deux minutes, l'eau colorée est montée de 14 cm par le simple effet de la capillarité dans l'échantillon traité contre 10 cm dans l'échantillon non traité.

## Revendications

1. Procédé d'obtention d'un matériau composite comprenant un renfort verrier dans une matrice organique thermoplastique choisie dans le groupe constitué par les polyvinylalcool, polyvinylacétate, polyvinylchlorure, polytétrafluoroéthylène et les copolymères dérivés de ces polymères, ledit renfort se présentant sous forme de mèches, grilles, voiles, tissés, non tissés ou similaires et présentant à sa surface un ensimage organique, ledit procédé comprenant au moins les étapes suivantes :
- traitement de surface du renfort verrier par un plasma homogène d'un mélange gazeux, dans une atmosphère gazeuse contrôlée permettant une oxydation ou une nitruration de l'ensimage organique présent à la surface du renfort verrier, ledit plasma homogène étant mis en oeuvre :
a) à une pression de l'ordre de 10⁻⁵ bar, ou
b) par un plasma à la pression atmosphérique, le gaz étant constitué majoritairement d'azote, seul ou en mélange avec des espèces réductrices, ou d'un gaz neutre choisi parmi l'hélium ou l'argon en mélange avec une ou plusieurs espèces oxydantes,
- imprégnation du renfort fonctionnalisé par une émulsion ou une suspension aqueuse de la matrice ou directement par la matrice sans étape intermédiaire telle que le dépôt d'un agent de couplage.

2. Procédé selon la revendication précédente, dans lequel la température du mélange gazeux est inférieure à 90°C.

3. Procédé selon l'une des revendications précédentes, dans lequel le mélange gazeux comprend ou est constitué d'oxygène.

4. Procédé selon l'une des revendications 1 ou 2, dans lequel le mélange gazeux comprend ou est constitué de N₂ ou d'un mélange de N₂ et d'un gaz réducteur du type H₂ ou NH₃.

5. Procédé selon l'une des revendications 1 ou 2, dans lequel le mélange gazeux comprend ou est constitué de NH₃.

6. Procédé selon l'une des revendications précédentes, dans lequel le renfort verrier est choisi parmi les tissus de verre, les non-tissés du type complexe, les voiles, les mats, les grilles de verre, notamment pour une application bâtiment, les fils unitaires ensimés ou préalablement transformés, les fils composés, le type de verre pouvant être choisi parmi les verres E, R, ECR, S, ou encore toute composition verrière connue pour ses propriétés anticorrosions vis-à-vis de conditions acides ou basiques, et/ou de haute résistance mécanique.

## Patentansprüche

1. Verfahren zum Erhalten eines Verbundwerkstoffs, umfassend eine Glasverstärkung in einer thermoplastischen organischen Matrix, ausgewählt aus der Gruppe, die von Polyvinylalkohol, Polyvinylacetat, Polyvinylchlorid, Polytetrafluorethylen und den von diesen Polymeren abgeleiteten Copolymeren gebildet ist, wobei die Verstärkung in Form von Fasersträngen, Gittern, Flor, Gewirken, Vliesstoffen oder dergleichen vorliegt und auf ihrer Oberfläche eine organische Schlichte aufweist, wobei das Verfahren mindestens die folgenden Schritte umfasst:
- Oberflächenbehandeln der Glasverstärkung mit einem homogenen Plasma eines Gasgemischs in einer kontrollierten Gasatmosphäre, die eine Oxidation oder eine Nitrierung der auf der Oberfläche der Glasverstärkung befindlichen organischen Schlichte erlaubt, wobei das homogene Plasma umgesetzt wird :
a) bei einem Druck von zirka 10⁻⁵ bar oder
b) durch ein Plasma bei atmosphärischem Druck, wobei das Gas mehrheitlich von Stickstoff, allein oder im Gemisch mit reduzierenden Spezies, oder von einem neutralen Gas, ausgewählt aus Helium oder Argon im Gemisch mit einer oder mehreren oxidierenden Spezies, gebildet ist,
- Imprägnieren der funktionalisierten Verstärkung durch eine Emulsion oder wässrige Suspension der Matrix oder direkt durch die Matrix ohne Zwischenschritt wie die Anlagerung eines Kopplungsmittels.

2. Verfahren nach vorangehendem Anspruch, wobei die Temperatur des Gasgemischs unter 90 °C liegt.

3. Verfahren nach einem der vorangehenden Ansprüche, wobei das Gasgemisch Sauerstoff umfasst oder davon gebildet ist.

4. Verfahren nach einem der Ansprüche 1 oder 2, wobei das Gasgemisch N₂ oder ein Gemisch aus N₂ und einem Reduktionsgas vom Typ H₂ oder NH₃ umfasst oder davon gebildet ist.

5. Verfahren nach einem der Ansprüche 1 oder 2, wobei das Gasgemisch NH₃ umfasst oder davon gebildet ist.

6. Verfahren nach einem der vorangehenden Ansprüche, wobei die Glasverstärkung aus den Glasgeweben, den Vliesstoffen komplexer Art, den Floren, den Matten, den Glasgittern, insbesondere für eine bauliche Anwendung, den geschlichteten oder im Vorfeld verarbeiteten Einzelfäden, den zusammengesetzten Fäden ausgewählt ist, wobei die Glasart aus den Glasarten E, R, ECR, S oder auch aus jeder wegen ihrer antikorrosiven Eigenschaften gegenüber sauren oder basischen Bedingungen und/oder hohen mechanischen Festigkeit bekannten Glaszusammensetzung ausgewählt sein kann.

## Claims

1. A process for obtaining a composite comprising a glass reinforcement or substrate in an organic or mineral matrix, said reinforcement or substrate being in the form of a roving, a scrim, a veil, a woven, a nonwoven or the like, comprising at least the following steps:
- surface treatment of the glass reinforcement with a homogeneous plasma of a gas mixture, in a controlled gaseous atmosphere allowing an oxidation or a nitriding of the organic size present on the surface of the glass reinforcement, said homogeneous plasma being employed:
a) at a pressure of the order of 10⁻⁵ bar or
b) substantially at atmospheric pressure, said gas consisting predominantly of nitrogen, by itself or mixed with reducing species or of an helium or argon inert gas, mixed with one or more oxidizing species,
- impregnation of the functionalized reinforcement with an aqueous emulsion or suspension of the matrix or directly with the matrix without an intermediate step such as the deposition of a coupling agent.

2. The process as claimed in the preceding claim, in which the temperature of the gas mixture is below 90°C.

3. The process as claimed in one of the preceding claims, in which the gas mixture comprises or consists of oxygen.

4. The process as claimed in claims 1 or 2, in which the gas mixture comprises or consists of N₂ or a mixture of N₂ and a reducing gas of the H₂ or NH₃ type.

5. The process as claimed in claims 1 or 2, in which the gas mixture comprises or consists of NH₃.

6. A process as claimed in one of the preceding claims, in which the glass reinforcement is chosen from glass wovens, nonwovens of complex type, veils, mats, glass scrims, especially for a building application, unitary strands sized or converted beforehand, and compound strands, it being possible for the type of glass to be chosen from E-glass, R-glass, ECR-glass and S-glass, or else any glass composition known for its corrosion-resistance properties under acid or basic conditions and/or for its high mechanical strength.
